# EUROPEAN PATENT APPLICATION

(11) **EP 4 571 417 A1**
(43) Date of publication of application: **18.06.2025**
(21) Application number: 23307214.9
(22) Date of filing: 14.12.2023
(51) Int. Cl.: G03F 7/027, G03F 7/029, G03F 7/031, C07D 311/04, C08F 2/50, C08F 22/32

(54) **PROCESS OF CURING ETHYLENICALLY UNSATURATED COMPOUNDS WITH COUMARIN THIOESTER PHOTOINITIATORS**

(71) Applicant: ARKEMA FRANCE, 92800 Puteaux (FR); Centre National de la Recherche Scientifique, 75016 Paris (FR); Université de Haute Alsace, 68093 Mulhouse Cedex (FR)
(72) Inventor: SQUIRES, Kelly, WETHERBY, LS22 7NS (GB); SEHNAL, Petr, WETHERBY, LS22 7NS (GB); PLENDERLEITH, Richard, WETHERBY, LS22 7NS (GB); GARRA, Patxi, 08193 BELLATERRA (ES); FAGGI, Enrico, 08193 BELLATERRA (ES); SPRICK, Élodie, 69110 SAINTE FOY LES LYON (FR); LALEVEE, Jacques, 68200 MULHOUSE (FR); BECHT, Jean-Michel, 68057 MULHOUSE (FR)
(74) Representative: Lavoix

(57) **Abstract**

The present invention relates to a process for curing under air one or more ethylenically unsaturated compounds with a coumarin thioester photoinitiator, preferably a photoinitiator activable under 250 to 550 nm, in particular 250 to 460 nm, light irradiation.

## Description

### TECHNICAL FIELD

The present invention relates to a process for curing under air one or more ethylenically unsaturated compounds with a coumarin thioester photoinitiator, preferably a photoinitiator activable under 250 to 550 nm, in particular 250 to 460 nm, light irradiation.

### TECHNICAL BACKGROUND

The increasing use of UV-visible curing technologies in areas such as high-speed printing, surface coating and additive manufacturing places high demands on the parameters of polymer networks that form in these processes. In particular, reactive photoinitiators are needed, that are thermally and chemically stable and that, after being activated by light or UV-radiation, can act as catalysts for a variety of anionic and/or free radical initiated polymerization reactions.

The most common photoinitiators are active under UV-B and UV-C light sources but there is a push to find photoinitiators active at longer wavelengths as the industry moves to using LED curing.

Photoinitiators which can be effectively activated by low-energy light sources such as LEDs without the need for additional sensitizers, but show high thermal stability in formulations before light exposure, are required.

Some common photoinitiators, such as (2,4,6-trimethylbenzoyldiphenylphosphine oxide (SpeedCure TPO), 2-Benzyl-2-dimethylamino-1-(4-morpholinophenyl)-butan-1-one (SpeedCure BDMB), 2-(dimethylamino)-2-[(4-methylphenyl)methyl]-1-[4-(morpholin-4-yl)phenyl]butan-1-one (Omnirad 379) and 2-hydroxy-2-methyl-1-phenylpropanone (SpeedCure 97), used for curing at longer wavelengths under LED (365 nm upwards) are subject to reclassification.
Commercially available Esacure 3644 (IGM) is a ketocoumarin that is able to cure under LED wavelengths, but because it is a Type II photoinitiator it requires an amine for hydrogen abstraction to do so.

A further serious disadvantage of common radical photoinitiators (for example phosphine oxides such as 2,4,6-trimethylbenzoyl diphenylphosphine oxide (TPO)) is their susceptibility to oxygen inhibition and subsequent inability to surface cure. This results in tacky coatings and excessive yellowing within the coating due to additional passes under the lamp.

Thus, there is a need for photoinitiators that can cure ethylenically unsaturated monomers without additional components, that can achieve a tack-free surface cure and/or that can display some levels of photobleaching.

There is also a particular need for an initiating system that is active under LED wavelengths and preferably does not suffer from oxygen inhibition.

Further, the photoinitiators and their photoproducts need to impart minimal coloration to the finished product and show low volatility and low toxicity.

CN 116693487 discloses coumarin-based thioester photoinitiators having an aryl or heteroaryl side chain. Said photoinitiators are disclosed as suitable for LED photopolymerization; however the curing process disclosed in said reference is carried out under inert atmosphere.

There is therefore a need for curing ethylenically unsaturated monomer formulations under a wide wavelength range of LED light in air, and for photoinitiators which show reduced or no oxygen inhibition effects.

### SUMMARY OF THE INVENTION

It is a first object of the invention to provide a process for curing one or more ethylenically unsaturated compounds, said process comprising:
- mixing one or more ethylenically unsaturated compounds with a compound of formula (I): 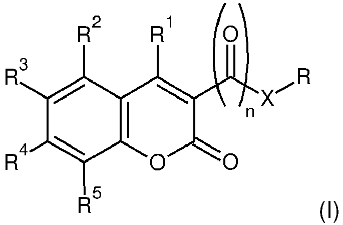 wherein n, R¹, R², R³, R⁴, R⁵, X and R are as defined herein;
   and
- irradiating said mixture with at least one light source under air.

It is a second object of the invention to provide a compound of formula (I'): 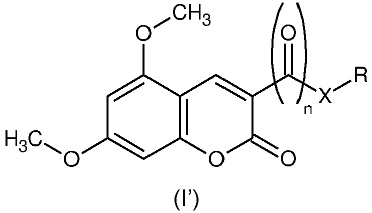 wherein n, X and R are defined herein.

In a third object, the invention also provides a compound selected from one of the following structures (1a) to (1 h):

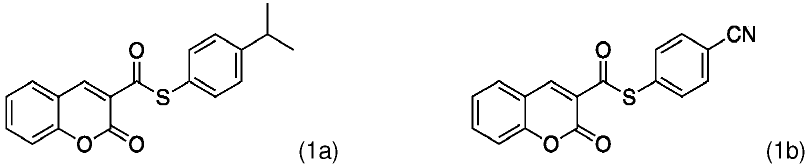

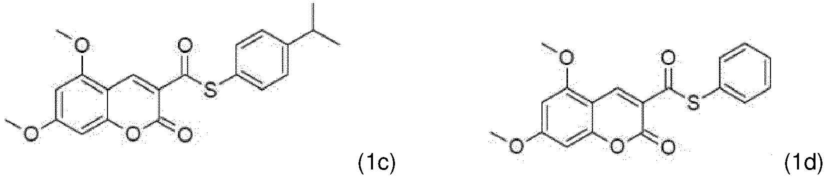

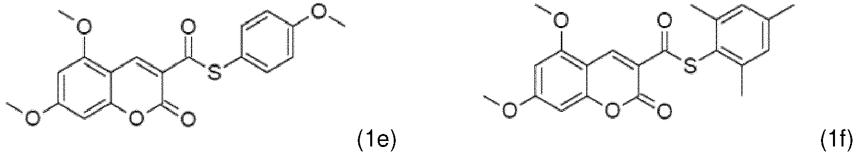

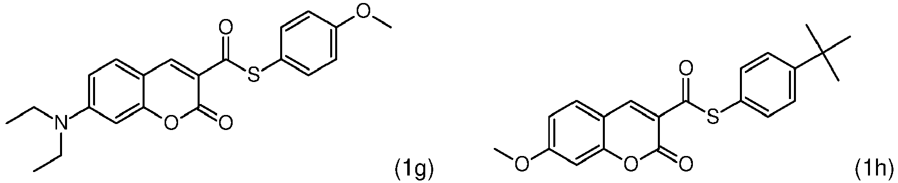

In a fourth object, the invention also provides the use of a compound according to the invention as a photoinitiator, in particular as a photoinitiator having photobleaching properties.

In a fifth object, the invention provides a process for the preparation of a compound according to the invention, the process comprising reacting a compound of formula (III) with a compound of formula (IV): 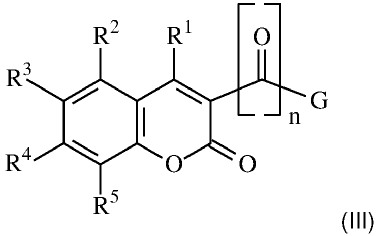 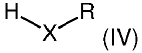 wherein n, R¹, R², R³, R⁴, R⁵, R, X and G are as defined herein.

In a sixth object, the invention provides a process for the preparation of a compound according to the invention, the process comprising reacting a compound of formula (VI) with a compound of formula (IV): 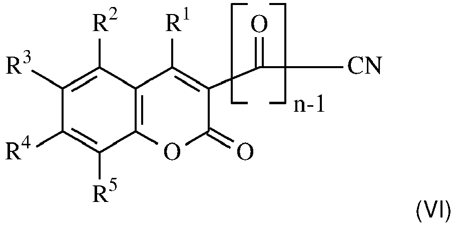 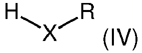 wherein n, R¹, R², R³, R⁴, R⁵, R and X are as defined herein.

In a seventh object, the invention provides a process for the preparation of a compound according to the invention, the process comprising reacting a compound of formula (VI) with hydrogen sulfide (H₂S) and a compound of formula (VII):

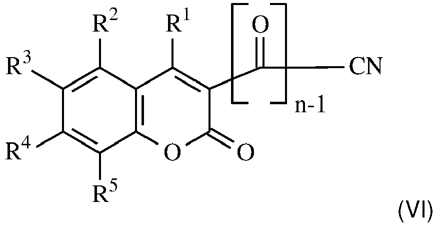

Hal―R (VII)

wherein n, R¹, R², R³, R⁴, R⁵, R and Hal are as defined herein.

### DETAILED DESCRIPTION

The invention will now be described in more detail without limitation in the following description.

### Definitions

In the present application, the term "comprise(s) a/an" means "comprise(s) one or more".

Unless mentioned otherwise, the % by weight in a compound or a composition are expressed based on the weight of the compound, respectively of the composition.

The term *"halogen"* means an atom selected from C!, Br, F and I.

The term *"alkyl"* means a monovalent saturated acyclic hydrocarbon group of formula -CₙH₂ₙ₊₁ wherein n is 1 to 200. An alkyl may be linear or branched. A « (C₁-C₂₀)alkyl » means an alkyl having 1 to 20 carbon atoms. Examples of alkyl groups include methyl, ethyl, propyl, isopropyl, butyl, isobutyl, tert-butyl, pentyl, hexyl, 2-methylbutyl, 2,2-dimethylpropyl, n-hexyl, 2-methylpentyl, 2,2-dimethylbutyl, n-heptyl, 2-ethylhexyl, and the like.

The term "*haloalkyl*" means an alkyl as defined above wherein at least one hydrogen atom is replaced by a halogen atom (i.e. Cl, Br, I or F).

The term *"perfluoroalkyl"* means an alkyl as defined above wherein all of the hydrogen atoms are replaced by a fluorine atom.

The term *"alkylene"* means a linker derived from an alkyl by removing one hydrogen for each point of attachment of the linker.

The term *"alkenyl"* means a monovalent acyclic hydrocarbon group comprising one or more carbon-carbon double bonds. An alkenyl may be linear or branched. Examples of alkenyl groups include ethenyl, propenyl, butenyl, pentenyl, hexenyl, octenyl, and the like.

The term *"aryl"* means an optionally substituted polyunsaturated aromatic group. The aryl may contain a single ring (i.e. phenyl) or more than one ring wherein at least one ring is aromatic. When the aryl comprises more than one ring, the rings may be fused, linked via a covalent bond (for example biphenyl). The aromatic ring may optionally comprise one to two additional fused rings. The term *"aryl"* also encompasses aromatic groups comprising one or more heteroatoms independently selected from N, O and S as ring atoms. The term *"aryl"* also encompasses partially hydrogenated derivatives of the carbocyclic system is described above. Examples of aryl groups include phenyl, naphthyl, biphenyl, phenanthrenyl, pyrenyl, naphthacenyl, pyrimidinyl, pyrazinyl, pyridazinyl, pyridinyl (including 2-aminopyridine), triazinyl, furanyl, quinolyl, isoquinolyl, quinoxalinyl, quinazolinyl, thienyl, imidazolyl, thiazolyl, indolyl (such as indol-3-yl), pyrryl, oxazolyl, benzofuranyl, benzothienyl, benzopyranyl, benzopyranonyl, benzodioxolyl, pyrazolyl, benzothiazolyl, isoxazolyl, triazolyl (including 1,2,4-triazole, 1,2,3-triazole, and 5-amino-1,2,4-triazole), tetrazolyl, indazolyl, isothiazolyl, 1,2,4-thiadiazolyl, purinyl, carbazolyl, isoxazolyl, benzimidazolyl, indolinyl, pyranyl, pyrazolyl, triazolyl, oxadiazolyl (including 1,2,3-oxadiazole, 1,2,4-oxadiazole, 1,2,5-oxadiazole, 3-amino-1,2,4-oxadiazole, 1,3,4-oxadiazole), thianthrenyl, indolizinyl, isoindolyl, isobenzofuranyl, pyrrolyl, benzoxazolyl, xanthenyl, 2H-pyrrolyl, 3H-indolyl, 4H-quinolizinyl, phthalazinyl, acridinyl, naphthyridinyl, quinazolinyl, phenanthridinyl, pymiridinyl, phenanthrolinyl, phenazinyl, isothiazolyl, phenothiazinyl, isoxazolyl, furazanyl, phenoxazinyl, xanthonyl, thioxanthenyl, thioxanthonyl, dibenzofuranyl, dibenzothienyl, acridinyl, acridonyl, phenoxathiinyl, chromanyl, isochromanyl, anthraquinonyl, naphthoquinonyl, dibenzodioxinyl, groups, and the like.

The term *"arylene"* means a linker derived from an aryl by removing one hydrogen for each point of attachment of the linker.

The term *"cycloalkyl"* means a monovalent non-aromatic alicyclic group comprising one or more cycles. The term cycloalkyl also encompasses non-aromatic cyclic groups comprising one or more heteroatoms independently selected from N, O and S as ring atoms. Examples of cycloalkyl groups include cyclopentyl, cyclohexyl, cycloheptyl, isobornyl, 1-decalin, norbornyl, adamantyl, oxiranyl, oxetanyl, tetrahydrofuranyl, tetrahydropyranyl, dioxanyl, pyrrolidinyl, piperidinyl, piperazinyl, morpholinyl, imidazolidinyl, oxazolidinyl, dioxolanyl, and the like.

The term *"cycloalkylene"* means a linker derived from a cycloalkyl by removing one hydrogen for each point of attachment of the linker.

The term *"alkoxy"* means a group of formula -O-alkyl, wherein the alkyl is as defined above.

The term *"aryloxy"* means a group of formula -O-aryl, wherein the aryl is as defined above.

The term *"linker"* means a plurivalent group. A linker may connect at least two moieties of a compound together, in particular 2 to 16 moieties of a compound together. For example, a linker that connects two moieties of a compound together is referred to as a divalent linker, a linker that connects three moieties of a compound together is referred to as a trivalent linker, etc....

The term *"hydrocarbon linker"* means a linker having a carbon backbone chain which may optionally be interrupted by one or more heteroatoms selected from N, O, S, Si and mixtures thereof. A hydrocarbon linker may be aliphatic, cycloaliphatic or aromatic. A hydrocarbon linker may be saturated or unsaturated. A hydrocarbon linker may be optionally substituted.

The term *"aliphatic"* means a non-aromatic acyclic compound. It may be linear or branched, saturated or unsaturated. It may be substituted by one or more groups, for example selected from alkyl, hydroxyl, halogen (Br, Cl, I, F), isocyanate, carbonyl, amine, carboxylic acid, -C(=O)-OR', -C(=O)-O-C(=O)-R', each R' being independently a C₁-C₂₀ alkyl. It may comprise one or more bonds selected from ether, ester, amide, urethane, urea and mixtures thereof.

The term *"acyclic"* means a compound that does not comprise any rings.

The term *"cycloaliphatic"* means a non-aromatic cyclic compound. It may be substituted by one or more groups as defined for the term *"aliphatic".* It may comprise one or more bonds as defined for the term *"aliphatic".*

The term *"aromatic"* means a compound comprising an aromatic ring, which means that respects Hückel's aromaticity rule, in particular a compound comprising a phenyl group. It may be substituted by one or more groups as defined for the term *"aliphatic".* It may comprise one or more bonds as defined for the term *"aliphatic".*

The term *"saturated"* means a compound that does not comprise any double or triple carbon-carbon bonds.

The term *"unsaturated"* means a compound that comprises a double or triple carbon-carbon bond, in particular a double carbon-carbon bond.

The term *"isocyanate group"* means a -N=C=O group.

As used herein "optionally substituted" refers to unsubstituted groups or groups substituted with one or more substituents identical or different.

Unless mentioned otherwise, optional substituents of alkyl and alkenyl groups can be independently selected from the group consisting of: -OH, -SH, halogen, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are independently selected from H, linear or branched -(C₁-C₂₀)alkyl and an optionally substituted -(C₅-C₁₂)aryl.

Unless mentioned otherwise, optional substituents of aryl and cycloalkyl groups can be independently selected from the group consisting of: -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are independently selected from H, linear or branched -(C₁-C₂₀)alkyl and an optionally substituted -(C₅-C₁₂)aryl.

### Curing process

The invention relates to a process for curing one or more ethylenically unsaturated compounds under air, said process comprising:
- mixing one or more ethylenically unsaturated compounds with a compound of formula (I): 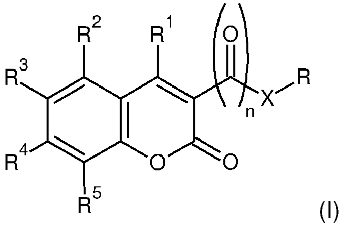 wherein n, R¹, R², R³, R⁴, R⁵, X and R are as defined hereinafter;
   and
- irradiating said mixture with at least one light source under air.

The light source is generally a LED (light-emitting diode) light source, or a broadband lamp with an optical filter that limits emission to wavelengths in the range of 250 to 550 nm, in particular 250 to 460 nm.

Preferably the light source is a LED light source, more preferably a LED light source having a maximum output wavelength in the range of 250 to 550 nm, in particular 250 to 460 nm. The maximum output wavelength of the LED light source may be from 350 to 450 nm, notably from 340 to 430 nm, even more preferably of 250 nm, 365 nm, 385 nm or 405 nm.

Since the photoactivity of the compounds of formula (I) is not altered by oxygen, it is not necessary to take specific precautions to prevent contact with oxygen during the curing process. Accordingly, the curing process is carried out under air. As used herein, the term "air" refers to a mixture of gases naturally present in the atmosphere, i.e a mixture of gases mainly constituted of nitrogen and oxygen. The molar fraction of oxygen in the air may be at least 10%, in particularly at least 15%, more particularly at least 20%. The molar fraction of nitrogen in the air may be at most 90%, in particular at most 85%, more particularly at most 80%.

The mixture containing the one or more ethylenically unsaturated compounds and the compound of formula (I) may be referred to as a curable composition. Such curable composition may further comprise one or more compounds conventionally used in curable compositions, such as:
- an anionic synergist;
- a radical photoinitiator;
- an acid stabilizer;
- a radical stabilizer,
- an additive; and
- a solvent.

The curable composition may be stationary when exposed to the light source. Alternatively, the curable composition may be in motion when exposed to the light source (for example, on a conveyor belt).

Curing may be accelerated or facilitated by supplying energy to the curable composition, such as by heating the curable composition. A curable composition may be partially cured by exposure to actinic radiation, with further curing being achieved by heating the partially cured article. For example, a product formed from the curable composition may be heated at a temperature of from 40°C to 120°C for a period of time of from 5 minutes to 12 hours.

Prior to curing, the curable composition may be applied to a substrate surface in any known conventional manner, for example, by spraying, jetting, knife coating, roller coating, casting, drum coating, dipping, and the like and combinations thereof. Indirect application using a transfer process may also be used. A substrate may be any commercially relevant substrate, such as a high surface energy substrate or a low surface energy substrate, such as a metal substrate or plastic substrate, respectively. The substrates may comprise metal, paper, cardboard, glass, thermoplastics such as polyolefins, polycarbonate, acrylonitrile butadiene styrene (ABS), and blends thereof, composites, wood, leather and combinations thereof. When used as an adhesive, the curable composition may be placed between two substrates and then cured, the cured composition thereby bonding the substrates together to provide an adhered article. Curable compositions in accordance with the present invention may also be formed or cured in a bulk manner (e.g., the curable composition may be cast into a suitable mold and then cured).

The process of the invention may be used to obtain a cured product. The cured product may be deemed as the reaction product of the curable composition, formed by curing. The cured product obtained with the process of the invention may be an ink, a coating, a sealant, an adhesive, a molded article or a 3D-printed article.

### Compound of formula (I)

The compound of formula (I) used in the curing process of the invention has the following formula: 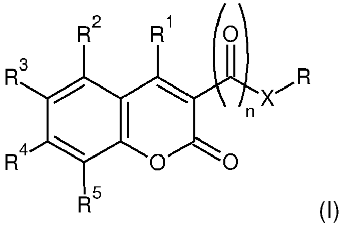 wherein
n is 1 or 2;
X represents S, Se or Te;
each R¹, R², R³, R⁴ and R⁵ is independently chosen from the group consisting of H, -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ; or two adjacent R², R³, R⁴ and R⁵ groups may form, with the carbon atoms to which they are attached, a 3 to 12 membered aliphatic ring or a 5 to 12 membered aromatic ring; wherein Rₐ, R_{b}, R_{d} and Rₑ identical or different are independently chosen from H and linear or branched (C₁-C₂₀)alkyl, and R_{c} is chosen from linear or branched -(C₁-C₂₀)alkyl and (C₅-C₁₂)aryl optionally substituted by one or more of -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -NR_{d}Rₑ, -NO₂ and -CN;
R is a (C₅-C₁₂)aryl optionally substituted by one or more groups chosen from OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above.

The preferred embodiments hereafter can be considered singly or combined with each other when applicable, and can be applied to formula (I) and any one of the formulae described hereafter:
According to an embodiment, R¹ is H and R², R³, R⁴ and R⁵ are independently chosen from the group consisting of H, linear or branched -O(C₁-C₂₀)alkyl and -NR_{d}Rₑ wherein R_{d} and Rₑ are independently a linear or branched -(C₁-C₂₀)alkyl.

According to an embodiment, R¹, R³ and R⁵ are H and R² and R⁴ are independently chosen from the group consisting of H, linear or branched -O(C₁-C₂₀)alkyl and -NR_{d}Rₑ wherein R_{d} and Rₑ are independently a linear or branched -(C₁-C₂₀)alkyl.

According to an embodiment, R¹, R², R³, R⁴ and R⁵ are H.

According to an embodiment, R¹, R², R³ and R⁵ are H and R⁴ is a linear or branched -O(C₁-C₂₀)alkyl, preferably methoxy.

According to an embodiment, R¹, R², R³ and R⁵ are H and R⁴ is -NR_{d}Rₑ wherein R_{d} and Rₑ are independently a linear or branched -(C₁-C₂₀)alkyl, preferably methyl or ethyl.

According to an embodiment, R², R³ and R⁵ are H and each of R¹ and R⁴ is independently a linear or branched -O(C₁-C₂₀)alkyl, preferably methoxy.

According to an embodiment, R¹, R³ and R⁵ are H and each of R² and R⁴ is independently a linear or branched -O(C₁-C₂₀)alkyl, preferably methoxy.

In formula (I), R is an optionally substituted (C₅-C₁₂)aryl. As used herein, the term "optionally substituted (C₅-C₁₂)aryl" means a (C₅-C₁₂)aryl which is optionally substituted by one or more groups chosen from OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above.

In particular, R may be a (C₅-C₁₂)aryl selected from phenyl, naphthyl, furanyl, thiophenyl, pyrrolyl and pyridinyl, all of which may be optionally substituted by one or more groups as defined above for the (C₅-C₁₂)aryl.

More particularly, R may be a phenyl or naphthyl, said phenyl or naphthyl being optionally substituted by one or more groups as defined above for the optionally substituted (C₅-C₁₂)aryl.

Even more particularly, R may be a phenyl or a naphthyl, said phenyl or naphthyl being optionally substituted by one or more groups selected from halogen, -CF₃, -NO₂, -CN, (C₅-C₁₂)aryl, linear or branched -(C₁-C₂₀)alkyl group, linear or branched -O(C₁-C₂₀)alkyl group, linear or branched -S(C₁-C₂₀)alkyl group, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above, preferably Rₐ, R_{b}, R_{c}, R_{d} and Rₑ are independently a linear or branched (C₁-C₂₀)alkyl.

More particularly still, R may be the residue of an aromatic monothiol (i.e the residue that is obtained by removing the SH group of an aromatic monothiol). Examples of suitable aromatic thiols, also referred to herein as M_{SH-Ar}, include thiophenol, 1- or 2-naphthalenethiol, 2-, 3- or 4-methylbenzenethiol, 2-, 3- or 4-isopropylbenzenethiol, 2-, 3- or 4-tert-butylbenzenethiol, 2-, 3- or 4-methoxybenzenethiol, 2-, 3- or 4-chlorobenzenethiol, 2-, 3- or 4-bromobenzenethiol, 2-, 3- or 4-fluorobenzenethiol, 2-, 3- or 4-nitrobenzenethiol, 2-, 3- or 4-cyanobenzenethiol, 2-, 3- or 4-trifluoromethylbenzenethiol, 2-, 3- or 4-methylthiobenzenethiol, methyl 2-, 3- or 4-mercaptobenzoate, 2,4,6-trimethylbenzenethiol, 2-, 3- or 4-(dimethylamino)benzenethiol and 2-, 3- or 4-(diethylamino)benzenethiol.

In formula (I), X represents S, Se or Te. In a preferred embodiment, X is S.

In formula (I), n is 1 or 2. In a preferred embodiment, n is 1.

In a particular embodiment, the compound of formula (I) is according to formula (I'): 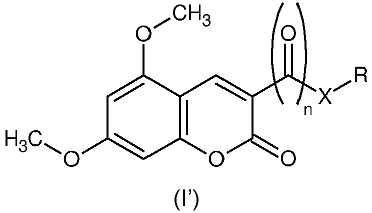 wherein n, X and R are defined as in formula (I).

In a particularly preferred embodiment, the compound of formula (I) is according to one of the following structures (1a) to (1h):

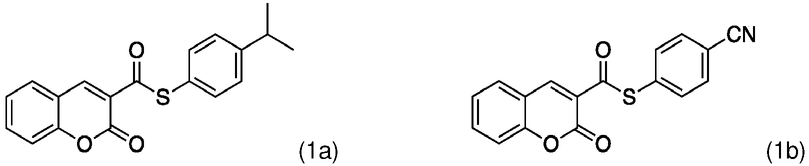

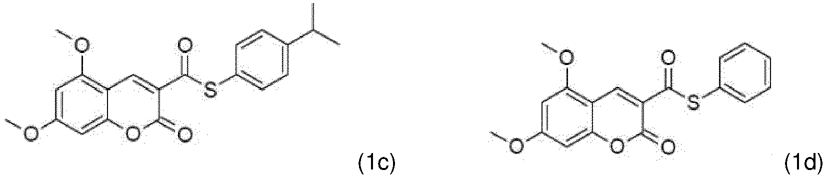

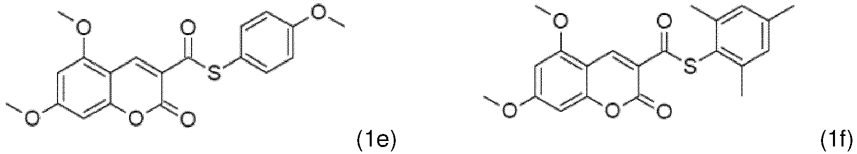

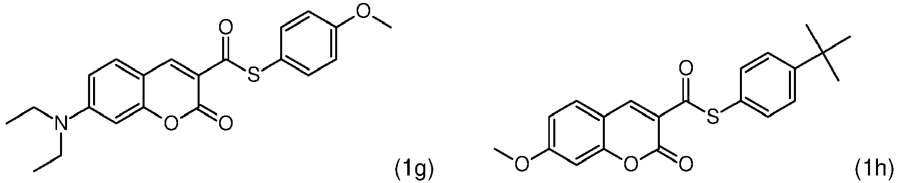

The amount of compound of formula (I) that is used in the process of the invention may be from 0.05% to 20%, in particular 0.10% to 15%, more particularly 0.15 % to 10%, even more particularly 0.20% to 5%, more particularly still 0.25% to 2%, by weight based on the total weight of the one or more ethylenically unsaturated compounds. If the process involves the use of a mixture of compounds of formula (I), the above weight percentage may be based on the weight of the mixture of compounds of formula (I).

### Ethylenically unsaturated compounds

The process of the invention involves curing (in other words polymerizing) one or more ethylenically unsaturated compounds.

As used herein, the term "ethylenically unsaturated compound" means a compound that comprises a polymerizable carbon-carbon double bond. A polymerizable carbon-carbon double bond is a carbon-carbon double bond that can react with another carbon-carbon double bond in a polymerization reaction. A polymerizable carbon-carbon double bond is generally comprised in a group selected from acrylate, methacrylate, cyanoacrylate, acrylamide, methacrylamide, styrene, maleate, fumarate, itaconate, methylene malonate, methylene acetyl acetonate, methylene beta-diketone, allyl, propenyl, vinyl and combinations thereof, preferably selected from acrylate, methacrylate, cyanoacrylate, allyl and vinyl, more preferably selected from acrylate, methacrylate and cyanoacrylate. The carbon-carbon double bonds of a phenyl ring are not considered as polymerizable carbon-carbon double bonds.

According to some preferred embodiments, the one or more ethylenically unsaturated compounds that are cured with the process of the invention comprise at least one of a cyanoacrylate-functionalized compound, a (meth)acrylate-functionalized compound and mixtures thereof.

The one or more ethylenically unsaturated compounds may comprise a cyanoacrylate. The one or more ethylenically unsaturated compounds may comprise a mixture of cyanoacrylates.

As used herein, the term "cyanoacrylate" refers to an organic compound which contains a carbon-carbon double bond, wherein one carbon atom involved in the carbon-carbon double bond is substituted with a cyano (-CN) group and an ester group.

Examples of suitable cyanoacrylates that may be cured with the process of the invention include methyl cyanoacrylate, ethyl cyanoacrylate, n-propyl cyanoacrylate, iso-propyl cyanoacrylate, *n*-butyl cyanoacrylate, *sec*-butyl cyanoacrylate, iso-butyl cyanoacrylate, tert-butyl cyanoacrylate, *n*-pentyl cyanoacrylate, 1-methylbutyl cyanoacrylate, 1-ethylpropyl cyanoacrylate, neopentyl cyanoacrylate, *n*-hexyl cyanoacrylate, 1-methylpentyl cyanoacrylate, *n*-heptyl cyanoacrylate, *n*-octyl cyanoacrylate, *2*-octyl cyanoacrylate, 2-ethylhexyl cyanoacrylate, *n*-nonyl cyanoacrylate, *n-*decyl cyanoacrylate, *n*-undecyl cyanoacrylate, *n*-dodecyl cyanoacrylate, n-octadecyl cyanoacrylate, allyl cyanoacrylate, cyclohexyl cyanoacrylate, 2-methoxyethyl cyanoacrylate, 2-ethoxyethyl cyanoacrylate, phenoxyethyl cyanoacrylate, 2-(1-alkoxy)propyl cyanoacrylate (for example 2-(1-methoxy)propyl cyanoacrylate), 2-(2'-alkoxy)-methoxyethyl-2"-cyanoacrylate (for example 2-(2'-methoxy)-methoxyethyl-2"-cyanoacrylate), 2-(2'-alkoxy)-ethoxyethyl-2"-cyanoacrylate (for example 2-(2'-methoxy)-ethoxyethyl-2"-cyanoacrylate, 2-(2'-ethoxy)-ethoxyethyl-2"-cyanoacrylate, 2-(2'-propyloxy)-ethoxyethyl-2"-cyanoacrylate, 2-(2'-butoxy)-ethoxyethyl-2"-cyanoacrylate, 2-(2'-pentyloxy)-ethoxyethyl-2"-cyanoacrylate or 2-(2'-hexyloxy)-ethoxyethyl-2"-cyanoacrylate), 2-(2'-alkoxy)-propyloxypropyl-2"-cyanoacrylate (for example 2-(2'-methoxy)-propyloxypropyl-2"-cyanoacrylate, 2-(2'-ethoxy)-propyloxypropyl-2"-cyanoacrylate, 2-(2'-propyloxy)-propyloxypropyl-2"-cyanoacrylate, 2-(2'-butyloxy)-propyloxypropyl-2"-cyanoacrylate, 2-(2'-pentyloxy)-propyloxypropyl-2"-cyanoacrylate, 2-(2'-hexyloxy)-propyloxypropyl-2"-cyanoacrylate), 2-(2'-alkoxy)-butyloxybutyl-2"-cyanoacrylate (for example 2-(2'-methoxy)-butyloxybutyl-2"-cyanoacrylate, 2-(2'-ethoxy)-butyloxybutyl-2"-cyanoacrylate, 2-(2'-butyloxy)-butyloxybutyl-2"-cyanoacrylate), 2-(3'-alkoxy)-propyloxyethyl-2"-cyanoacrylate for example 2-(3'-methoxy)-propyloxyethyl-2"-cyanoacrylate), 2-(3'-alkoxy)-butyloxyethyl-2"-cyanoacrylate (for example 2-(3'-methoxy)-butyloxyethyl-2"-cyanoacrylate), 2-(3'-alkoxy)-propyloxypropyl-2"-cyanoacrylate (for example 2-(3'-methoxy)-propyloxypropyl-2"-cyanoacrylate), 2-(3'-alkoxy)-butyloxypropyl-2"-cyanoacrylate (for example 2-(3'-methoxy)-butyloxypropyl-2"-cyanoacrylate 2-(2'-alkoxy)-ethoxypropyl-2"-cyanoacrylate (for example 2-(2'-methoxy)-ethoxypropyl-2"-cyanoacrylate), 2-(2'-alkoxy)-ethoxybutyl-2"-cyanoacrylate (for example 2-(2'-methoxy)-ethoxybutyl-2"-cyanoacrylate), trimethylsilyethyl cyanoacrylate, trimethylsilypropyl cyanoacrylate, tetrahydrofurfuryl cyanoacrylate, 1,6-hexanediol bis-cyanoacrylate, trimethylsilyloxyethyl cyanoacrylate triethylsilyloxyethyl cyanoacrylate, phenylethyl cyanoacrylate, and mixtures thereof.

More preferably, the cyanoacrylate is a mono-functional cyanoacrylate, more preferably selected from 2-methoxyethyl cyanoacrylate, 2-ethoxyethyl cyanoacrylate, methyl cyanoacrylate, ethyl cyanoacrylate, n-propyl cyanoacrylate, n-heptyl cyanoacrylate, n-octyl cyanoacrylate, iso-propyl cyanoacrylate or mixtures thereof.

When the one or more ethylenically unsaturated compounds comprise a cyanoacrylate, the amount of cyanoacrylate may be from 70% to 99% by weight, preferably from 75% to 95% by weight, and more preferably from 70 to 90% by weight based on the total weight of the one or more ethylenically unsaturated compounds.

The one or more ethylenically unsaturated compounds may comprise a (meth)acrylate-functionalized compound. The one or more ethylenically unsaturated compounds may comprise a mixture of (meth)acrylate-functionalized compounds.

As used herein, the term "(meth)acrylate-functionalized compound" means a compound comprising a (meth)acrylate group, in particular an acrylate group. The term "(meth)acrylate-functionalized compound" here encompasses containing more than one (meth)acrylate group, such as 2, 3, 4, 5 or 6 (meth)acrylate groups, commonly referred to as "oligomers" comprising a (meth)acrylate group. The term "(meth)acrylate group" encompasses acrylate groups (-O-CO-CH=CH₂) and methacrylate groups (-O-CO-C(CH₃)=CH₂).

In one embodiment, the one or more ethylenically unsaturated compounds may contain from 40% to 99%, from 45% to 90%, from 50% to 85%, or from 50% to 80% by weight of (meth)acrylate-functionalized compounds based on the total weight of the one or more ethylenically unsaturated compounds.

Alternatively, the one or more ethylenically unsaturated compounds may contain from 5% to 50%, from 10% to 45%, from 15% to 40% or from 15% to 30%, by weight of (meth)acrylate-functionalized compounds based on the total weight of the one or more ethylenically unsaturated compounds.

The (meth)acrylate-functionalized compound may be selected from a (meth)acrylate-functionalized monomer, a (meth)acrylate-functionalized oligomer and mixtures thereof.

As used herein, the term "(meth)acrylate-functionalized monomer" means a monomer comprising a (meth)acrylate group, in particular an acrylate group. The term "(meth)acrylate-functionalized oligomer" means an oligomer comprising a (meth)acrylate group, in particular an acrylate group.

In one embodiment, the one or more ethylenically unsaturated compounds comprise a (meth)acrylate-functionalized monomer. The one or more ethylenically unsaturated compounds may comprise a mixture of (meth)acrylate-functionalized monomers.

The (meth)acrylate-functionalized monomer may have a molecular weight of less than 600 g/mol, in particular from 100 to 550 g/mol, more particularly 200 to 500 g/mol.

The (meth)acrylate-functionalized monomer may have 1 to 6 (meth)acrylate groups, in particular 1 to 3 (meth)acrylate groups.

The (meth)acrylate-functionalized monomer may comprise a mixture of (meth)acrylate-functionalized monomers having different functionalities. For example, the (meth)acrylate-functionalized monomer may comprise a mixture of a (meth)acrylate-functionalized monomer containing a single acrylate or methacrylate group per molecule (referred to herein as "mono(meth)acrylate-functionalized compounds") and a (meth)acrylate-functionalized monomer containing 2 or more, preferably 2 or 3, acrylate and/or methacrylate groups per molecule.

In one embodiment, the (meth)acrylate functionalized monomer comprises a mono(meth)acrylate-functionalized monomer. The mono(meth)acrylate-functionalized monomer may advantageously function as a reactive diluent and reduce the viscosity of the composition of the invention.

Examples of suitable mono(meth)acrylate-functionalized monomers that may be cured with the process of the invention include, but are not limited to, mono-(meth)acrylate esters of aliphatic alcohols (wherein the aliphatic alcohol may be straight chain, branched or alicyclic and may be a mono-alcohol, a di-alcohol or a polyalcohol, provided only one hydroxyl group is esterified with (meth)acrylic acid); mono-(meth)acrylate esters of aromatic alcohols (such as phenols, including alkylated phenols); mono-(meth)acrylate esters of alkylaryl alcohols (such as benzyl alcohol); mono-(meth)acrylate esters of oligomeric and polymeric glycols such as diethylene glycol, triethylene glycol, dipropylene glycol, tripropylene glycol, polyethylene glycol, and polypropylene glycol); mono-(meth)acrylate esters of monoalkyl ethers of glycols and oligoglycols; mono-(meth)acrylate esters of alkoxylated (e.g., ethoxylated and/or propoxylated) aliphatic alcohols (wherein the aliphatic alcohol may be straight chain, branched or alicyclic and may be a mono-alcohol, a di-alcohol or a polyalcohol, provided only one hydroxyl group of the alkoxylated aliphatic alcohol is esterified with (meth)acrylic acid); mono-(meth)acrylate esters of alkoxylated (e.g., ethoxylated and/or propoxylated) aromatic alcohols (such as alkoxylated phenols); caprolactone mono(meth)acrylates; and the like.

The following compounds are specific examples of mono(meth)acrylate-functionalized monomers that may be cured with the process of the present invention: methyl (meth)acrylate; ethyl (meth)acrylate; n-propyl (meth)acrylate; n-butyl (meth)acrylate; isobutyl (meth)acrylate; n-hexyl (meth)acrylate; 2-ethylhexyl (meth)acrylate; n-octyl (meth)acrylate; isooctyl (meth)acrylate; n-decyl (meth)acrylate; n-dodecyl (meth)acrylate; tridecyl (meth)acrylate; tetradecyl (meth)acrylate; hexadecyl (meth)acrylate; 2-hydroxyethyl (meth)acrylate; 2- and 3-hydroxypropyl (meth)acrylate; 2-methoxyethyl (meth)acrylate; 2-ethoxyethyl (meth)acrylate; 2- and 3-ethoxypropyl (meth)acrylate; tetrahydrofurfuryl (meth)acrylate; alkoxylated tetrahydrofurfuryl (meth)acrylate; 2-(2-ethoxyethoxy)ethyl (meth)acrylate; cyclohexyl (meth)acrylate; glycidyl (meth)acrylate; isodecyl (meth)acrylate; lauryl (meth)acrylate; 2-phenoxyethyl (meth)acrylate; alkoxylated phenol (meth)acrylates; alkoxylated nonylphenol (meth)acrylates; cyclic trimethylolpropane formal (meth)acrylate; isobornyl (meth)acrylate; tricyclodecanemethanol (meth)acrylate; tert-butylcyclohexanol (meth)acrylate; trimethylcyclohexanol (meth)acrylate; diethylene glycol monomethyl ether (meth)acrylate; diethylene glycol monoethyl ether (meth)acrylate; diethylene glycol monobutyl ether (meth)acrylate; triethylene glycol monoethyl ether (meth)acrylate; ethoxylated lauryl (meth)acrylate; methoxy polyethylene glycol (meth)acrylates; hydroxyl ethyl-butyl urethane (meth)acrylates; 3-(2-hydroxyalkyl)oxazolidinone (meth)acrylates; and combinations thereof.

In one embodiment, the (meth)acrylate functionalized monomer may comprise a (meth)acrylate-functionalized monomer containing two or more (meth)acrylate groups per molecule.

Examples of suitable (meth)acrylate-functionalized monomers containing two or more (meth)acrylate groups per molecule include acrylate and methacrylate esters of polyhydric alcohols (organic compounds containing two or more, e.g., 2 to 6, hydroxyl groups per molecule). Specific examples of suitable polyhydric alcohols include C2-20 alkylene glycols (glycols having a C2-10 alkylene group may be preferred, in which the carbon chain may be branched; e.g., ethylene glycol, trimethylene glycol, 1,2-propylene glycol, 1,2-butanediol, 1,3-butanediol, 2,3-butanediol, tetramethylene glycol (1,4-butanediol), 1,5-pentanediol, 1,6-hexanediol, 1,8-octanediol, 1,9-nonanediol, 1,12-dodecanediol, cyclohexane-1,4-dimethanol, bisphenols, and hydrogenated bisphenols, as well as alkoxylated (e.g., ethoxylated and/or propoxylated) derivatives thereof), diethylene glycol, glycerin, alkoxylated glycerin, triethylene glycol, dipropylene glycol, tripropylene glycol, trimethylolpropane, alkoxylated trimethylolpropane, ditrimethylolpropane, alkoxylated ditrimethylolpropane, pentaerythritol, alkoxylated pentaerythritol, dipentaerythritol, alkoxylated dipentaerythritol, cyclohexanediol, alkoxylated cyclohexanediol, cyclohexanedimethanol, alkoxylated cyclohexanedimethanol, norbornene dimethanol, alkoxylated norbornene dimethanol, norbornane dimethanol, alkoxylated norbornane dimethanol, polyols containing an aromatic ring, cyclohexane-1,4-dimethanol ethylene oxide adducts, bis-phenol ethylene oxide adducts, hydrogenated bisphenol ethylene oxide adducts, bisphenol propylene oxide adducts, hydrogenated bisphenol propylene oxide adducts, cyclohexane-1,4-dimethanol propylene oxide adducts, sugar alcohols and alkoxylated sugar alcohols. Such polyhydric alcohols may be fully or partially esterified (with (meth)acrylic acid, (meth)acrylic anhydride, (meth)acryloyl chloride or the like), provided they contain at least two (meth)acrylate functional groups per molecule.

Exemplary (meth)acrylate-functionalized monomers containing two or more (meth)acrylate groups per molecule may include bisphenol A di(meth)acrylate; hydrogenated bisphenol A di(meth)acrylate; ethylene glycol di(meth)acrylate; diethylene glycol di(meth)acrylate; triethylene glycol di(meth)acrylate; tetraethylene glycol di(meth)acrylate; polyethylene glycol di(meth)acrylate; propylene glycol di(meth)acrylate; dipropylene glycol di(meth)acrylate; tripropylene glycol di(meth)acrylate; tetrapropylene glycol di(meth)acrylate; polypropylene glycol di(meth)acrylate; polytetramethylene glycol di(meth)acrylate; 1,2-butanediol di(meth)acrylate; 2,3-butanediol di(meth)acrylate; 1,3-butanediol di(meth)acrylate; 1,4-butanediol di(meth)acrylate; 1,5-pentanediol di(meth)acrylate; 1,6-hexanediol di(meth)acrylate; 1,8-octanediol di(meth)acrylate; 1,9-nonanediol di(meth)acrylate; 1,10-nonanediol di(meth)acrylate; 1,12-dodecanediol di(meth)acrylate; neopentyl glycol di(meth)acrylate; 2-methyl-2,4-pentanediol di(meth)acrylate; polybutadiene di(meth)acrylate; cyclohexane-1,4-dimethanol di(meth)acrylate; tricyclodecane dimethanol di(meth)acrylate; metallic di(meth)acrylates; modified metallic di(meth)acrylates; glyceryl di(meth)acrylate; glyceryl tri(meth)acrylate; trimethylolethane tri(meth)acrylate; trimethylolethane di(meth)acrylate; trimethylolpropane tri(meth)acrylate; trimethylolpropane di(meth)acrylate; pentaerythritol di(meth)acrylate; pentaerythritol tri(meth)acrylate; pentaerythritol tetra(meth)acrylate, di(trimethylolpropane) diacrylate; di(trimethylolpropane) triacrylate; di(trimethylolpropane) tetraacrylate, sorbitol penta(meth)acrylate; di(pentaerythritol) tetraacrylate; di(pentaerythritol) pentaacrylate; di(pentaerythritol) hexa(meth)acrylate; tris (2-hydroxyethyl) isocyanurate tri(meth)acrylate; as well as the alkoxylated (e.g., ethoxylated and/or propoxylated) derivatives thereof; and combinations thereof.

The one or more ethylenically unsaturated compounds cured by the process of the invention may comprise 0 to 99.5%, in particular 5 to 90%, more particularly 10 to 80%, even more particularly 15 to 75%, more particularly still 20 to 70% by weight of (meth)acrylate-functionalized monomer based on the total weight of the one or more ethylenically unsaturated compounds. In particular, the one or more ethylenically unsaturated compounds cured by the process of the invention may comprise 5 to 50% or 10 to 50% or 15 to 50% or 20 to 50% or 25 to 50% or 30 to 50%, by weight of (meth)acrylate-functionalized monomer based on the total weight of the one or more ethylenically unsaturated compounds. Alternatively, the one or more ethylenically unsaturated compounds cured by the process of the invention may comprise 50 to 99.5% or 55 to 99.5% or 60 to 99.5% or 65 to 99.5% or 70 to 99.5%, by weight of (meth)acrylate-functionalized monomer based on the total weight of the one or more ethylenically unsaturated compounds.

In one embodiment, the one or more ethylenically unsaturated compounds comprise a (meth)acrylate-functionalized oligomer. The one or more ethylenically unsaturated compounds may comprise a mixture of (meth)acrylate-functionalized oligomers.

The (meth)acrylate-functionalized oligomer may be selected in order to enhance the flexibility, strength and/or modulus, among other attributes, of a cured product obtained with the process of the present invention.

The (meth)acrylate functionalized oligomer may have 1 to 18 (meth)acrylate groups, in particular 2 to 6 (meth)acrylate groups, more particularly 2 to 6 acrylate groups.

The (meth)acrylate functionalized oligomer may have a number average molecular weight equal or more than 600 g/mol, in particular 800 to 15,000 g/mol, more particularly 1,000 to 5,000 g/mol.

In particular, the (meth)acrylate-functionalized oligomers may be selected from the group consisting of epoxy (meth)acrylates, polyester (meth)acrylates, polyether (meth)acrylates, urethane (meth)acrylates, (meth)acrylated poly(meth)acrylates and mixtures thereof.

Non-limiting examples of epoxy (meth)acrylates are the reaction products of an epoxide (such as glycidyl ethers, glycidyl esters, cycloaliphatic epoxides or epoxides obtained by epoxidation of mono- and/or polyunsaturated compounds) with a (meth)acrylating agent (such as (meth)acrylic acid, (meth)acrylic anhydride, (meth)acryloyl chloride or combinations thereof). The epoxide may be selected from 1,2,3,4-diepoxybutane; 1,2,4,5-diepoxypentane; 1,2,5,6-diepoxyhexane; 1,2,7,8-diepoxyoctane; 1,2,9,10-diepoxydecane; bisphenol A diglycidyl ether, bisphenol F diglycidyl ether, bisphenol S diglycidyl ether, brominated bisphenol A diglycidyl ether, brominated bisphenol F diglycidyl ether, brominated bisphenol S diglycidyl ether, epoxy novolak resin, hydrogenated bisphenol A diglycidyl ether, hydrogenated bisphenol F diglycidyl ether, hydrogenated bisphenol S diglycidyl ether, 3,4-epoxycyclohexylmethyl-3',4'-epoxycyclohexanecarboxylate, 2-(3,4-epoxycyclohexyl-5,5-spiro-3,4-epoxy)cyclohexane-1,4-dioxane, bis(3,4-epoxycyclohexylmethyl)adipate, vinylcyclohexene oxide, 4-vinylepoxycyclohexane, bis(3,4-epoxy-6-methylcyclohexylmethyl)adipate,3,4-epoxy-6-methylcyclohexy 1-3',4'-epoxy-6'-methylcyclohexanecarboxylate, methylenebis(3,4-epoxycyclohexane), dicyclopentadiene diepoxide, di(3,4-epoxycyclohexylmethyl) ether of ethylene glycol, ethylenebis(3, 4-epoxycyclohexanecarboxylate), ethylene glycol diglycidyl ether, 1,2- or 1,3-propylene glycol diglycidyl ether, 1,2-, 1,3- or 1,4-butanediol diglycidyl ether, 1,5-pentanediol diglycidyl ether, 1,6-hexanediol diglycidyl ether, 1,7-hexanediol diglycidyl ether, 1,8-octanediol diglycidyl ether, 1,9-nonanediol diglycidyl ether, 1,10-decanediol diglycidyl ether, 1,12-dodecanediol diglycidyl ether, 2-methyl-1,3-propanediol diglycidyl ether, neopentyl glycol diglycidyl ether, 2,2-diethyl-1,3-propane diol diglycidyl ether, 3-methyl-1,5-pentanediol diglycidyl ether, 3,3-dimethyl-1,5-pentanediol diglycidyl ether, 2,4-diethyl-1,5-pentanediol diglycidyl ether, 3,3-butylethyl-1,5-pentane diol diglycidyl ether, di-, tri- or tetra(ethylene glycol) diglycidyl ether, di-, tri- or tetra(1,2-propylene glycol) diglycidyl ether, di-, tri- or tetra(1,3-propylene glycol) diglycidyl ether, di-, tri- or tetra(1,4-butylene glycol) diglycidyl ether, a polyethylene glycol) diglycidyl ether, a polypropylene glycol) diglycidyl ether, a poly(trimethylene glycol) diglycidyl ether, a poly(tetramethylene glycol) diglycidyl ether, a polyethylene glycol-co-propylene glycol) diglycidyl ether, glycerol triglycidyl ether, a polyglycerol polyglycidyl ether, trimethylolmethane triglycidyl ether, trimethylolethane triglycidyl ether, trimethylolpropane triglycidyl ether, di(trimethylolpropane) tetraglycidyl ether, pentaerythritol tetraglycidyl ether, diglycidyl cyclohexanedicarboxylate, cyclohexane diglycidyl ether, cyclohexane-1,4-dimethanol diglycidyl ether, tricyclodecane dimethanol diglycidyl ether, isosorbide diglycidyl ether, pyrocatechol diglycidyl ether, resorcinol diglycidyl ether, cardol diglycidyl ether, phloroglucinol triglycidyl ether, pyrogallol triglycidyl ether, tris(hydroxyphenyl)methane triglycidyl ether, tris(hydroxyphenyl)ethane triglycidyl ether, diglycidyl phthalate, diglycidyl terephthalate, diglycidyl isophthalate, polyglycidyl ethers of a polyether polyol obtained by the addition of one or more alkylene oxides to an aliphatic polyhydric alcohol such as ethylene glycol, propylene glycol, and glycerol, diglycidyl esters of aliphatic long-chain (C6-C22) dibasic acids, monoglycidyl ethers of aliphatic higher alcohols, monoglycidyl ethers of phenol, cresol, butyl phenol, or polyether alcohols obtained by the addition of alkylene oxide to these compounds, glycidyl esters of higher fatty acids, an epoxidized vegetable oil (such as epoxidized soybean oil and epoxidized linseed oil), epoxybutylstearic acid, epoxyoctylstearic acid, epoxidized polybutadiene, triglycidyl isocyanurate and the like.

Non-limiting examples of polyester (meth)acrylates are the reaction products of a hydroxyl group-terminated polyester polyol with a (meth)acrylating agent (such as (meth)acrylic acid, (meth)acrylic anhydride, (meth)acryloyl chloride or combinations thereof). The reaction process may be conducted such that a significant concentration of residual hydroxyl groups remain in the polyester (meth)acrylate or may be conducted such that all or essentially all of the hydroxyl groups of the polyester polyol have been (meth)acrylated. The polyester polyols can be made by polycondensation reactions of a polyhydroxyl functional component (in particular a diol) and a polycarboxylic acid functional compound (in particular, a dicarboxylic acid or anhydride). To prepare the polyester (meth)acrylates, the hydroxyl groups of the polyester polyol are then partially or fully esterified by reacting with the (meth)acrylating agent. Polyester (meth)acrylates may also be synthesized by reacting a hydroxyl-containing (meth)acrylate such as a hydroxyalkyl (meth)acrylate (e.g., hydroxyethyl acrylate) with a polycarboxylic acid. The polyhydroxyl functional and polycarboxylic acid functional components can each have linear, branched, cycloaliphatic or aromatic structures and can be used individually or as mixtures.

Non-limiting examples of polyether (meth)acrylates are the condensation reaction products of a polyether polyol with a (meth)acrylating agent (such as (meth)acrylic acid, (meth)acrylic anhydride, (meth)acryloyl chloride or combinations thereof). Suitable polyether polyols can be linear or branched substances containing ether bonds and terminal hydroxyl groups. Polyether polyols can be prepared by ring opening polymerization of epoxides and other oxygen-containing heterocyclic compounds (e.g., ethylene oxide, 1,2-propylene oxide, butene oxide, tetrahydrofuran and combinations thereof) with a starter molecule. Suitable starter molecules include water, hydroxyl functional materials, polyester polyols and amines. Polyetherols may also be obtained by the condensation of diols such as glycols.

Non-limiting examples of urethane (meth)acrylates are the condensation reaction products of at least one polyisocyanate (e.g., diisocyanate, triisocyanate), at least one polyol (such as a polyether polyol or a polyester polyol) and a hydroxyl-functionalized (meth)acrylate (such as 2-hydroxyethyl (meth)acrylate or 3-hydroxypropyl (meth)acrylate) to provide terminal (meth)acrylate groups. For example, the urethane (meth)acrylate may contain two, three, four or more (meth)acrylate groups per molecule. The order of addition of the components to prepare the urethane (meth)acrylate is well known in the art. For example, the hydroxyl-functionalized (meth)acrylate may be first reacted with the polyisocyanate to obtain an isocyanate-functionalized (meth)acrylate, which is then reacted with the polyol. In yet another embodiment, the polyisocyanate may be first reacted with the polyol to obtain an isocyanate-functionalized polyol, which is thereafter reacted with a hydroxyl-functionalized (meth)acrylate. Alternatively, all the components may be combined and reacted at the same time.

Non-limiting examples of (meth)acrylated poly(meth)acrylates are substances having an oligomeric (meth)acrylic backbone which is functionalized with one or (meth)acrylate groups (which may be at a terminus of the oligomer or pendant to the acrylic backbone). The (meth)acrylic backbone may be a homopolymer, random copolymer or block copolymer comprised of repeating units of (meth)acrylic monomers. The (meth)acrylic monomers may be any monomeric (meth)acrylate such as C₁-C₆ alkyl (meth)acrylates as well as functionalized (meth)acrylates such as (meth)acrylates bearing hydroxyl, carboxylic acid and/or epoxy groups. (Meth)acrylated poly(meth)acrylates may be prepared using any procedures known in the art, such as by oligomerizing (meth)acrylic monomers, at least a portion of which are functionalized with hydroxyl, carboxylic acid and/or epoxy groups (e.g., hydroxyalkyl(meth)acrylates, (meth)acrylic acid, glycidyl (meth)acrylate) to obtain a functionalized poly(meth)acrylate, which is then reacted with one or more (meth)acrylate-containing reactants to introduce the desired (meth)acrylate functional groups.

### Other compounds conventionally used in curable compositions

The process of the invention may further comprise mixing one or more compounds conventionally used in curable compositions with said compound of formula (I) and said one or more ethylenically unsaturated compounds. Examples of compounds conventionally used in curable compositions include:
- an anionic synergist;
- a radical photoinitiator;
- an acid stabilizer;
- a radical stabilizer,
- an additive; and
- a solvent.

Such compounds may be as described below.

### Anionic synergist

The process of the invention may further comprise mixing an anionic synergist with said compound of formula (I) and said one or more ethylenically unsaturated compounds. Said anionic synergist may be present when the process is used to cure one or more ethylenically unsaturated compounds comprising a cyanoacrylate-functionalized compound, in particular to enhance the cure rate of said cyanoacrylate-functionalized compound.

In particular, the anionic synergist may be a metallocene.

Preferred metallocenes for use in the present invention include metallocenes in which each of the aromatic electron system ligands in the metallocene is a π-arene, indenyl, or η⁵-cyclopentadienyl ligand. The metallocene may be based on a transition metal selected from the group consisting of iron, osmium, and ruthenium. Iron-containing metallocenes, i.e., ferrocenes, are employed in certain aspects of the invention. However, in other embodiments, the metallocene compound may be an osmocene or a ruthenocene. Combinations of two or more different metallocene compounds may be used.

More particularly, the anionic synergist may be a ferrocene.

A ferrocene may be according to the following formula (VI): 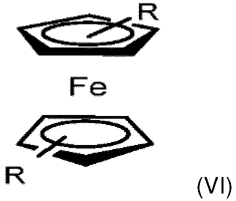 wherein each R is independently hydrogen or a linear or branched C₁-C₄ alkyl.

Specific examples of metallocenes useful in the present invention are disclosed in US 6503959, which is incorporated herein by reference.

The amount of anionic synergist may be from 50 to 1000 ppm based on the weight of the one or more ethylenically unsaturated compounds.

### Acid Stabilizer

The process of the invention may further comprise mixing an acid stabilizer with said compound of formula (I) and said one or more ethylenically unsaturated compounds. Said acid stabilizer may be present when the process is used to cure one or more ethylenically unsaturated compounds comprising a cyanoacrylate-functionalized compound, in particular to prevent premature curing.

In particular, the acid stabilizer may be a Lewis acid. Examples of suitable Lewis acid include of boron trifluoride, boron trifluoride etherate complexes (e.g., boron trifluoride diethyl etherate complex), boron trifluoride dihydrate, trimethylsilyl triflate, sulphur dioxide, sulphur trioxide, nitrogen oxide and mixtures thereof, most preferably from boron trifluoride etherate complexes.

Other types of suitable acid stabilizers include protic acids such as hydrogen halides (e.g., hydrogen fluoride) and sulfonic acids (e.g., p-toluenesulfonic acid).

The amount of acid stabilizer may be from 1 to 1000 ppm, e.g. from 10 to 100 ppm based on the total weight of the one or more ethylenically unsaturated compounds.

### Radical photoinitiator

The process of the invention may further comprise mixing a radical photoinitiator with said compound of formula (I) and said one or more ethylenically unsaturated compounds. The radical photoinitiator may be a radical photoinitiator having Norrish type I activity and/or Norrish type II activity, more particularly a radical photoinitiator having Norrish type I activity. The radical photoinitiator is distinct from the compound of formula (I).

Non-limiting types of radical photoinitiators suitable for use in the process of the invention include, for example, benzoins, benzoin ethers, acetophenones, α-hydroxy acetophenones, benzil, benzil ketals, phosphine oxides, acylphosphine oxides, α-hydroxyketones, phenylglyoxylates, α-aminoketones, benzoyl formates, acylgermanyl compounds, polymeric derivatives thereof, and mixtures thereof., but are not limited to, benzoin, benzoin methyl ether, benzoin ethyl ether, benzoin isopropyl ether, alpha-methylbenzoin, alpha-phenylbenzoin, Michler's ketone, 1-hydroxyphenyl ketones, acetophenone, 2,2-diethyloxyacetophenone, benzil, α-hydroxyketone, 2,4,6-trimethylbenzoyldiphenyl phosphine oxide, 2,2-dimethoxy-1,2-phenylacetophenone, 1-hydroxycyclohexyl phenyl ketone, 2-methyl-1-[4-(methylthio) phenyl]-2-morpholinopropanone, 2-hydroxy-2-methyl-1-phenyl-propanone, oligomeric α-hydroxy ketone, benzoyl phosphine oxides, phenylbis(2,4,6-trimethylbenzoyl)phosphine oxide, ethyl(2,4,6-trimethylbenzoyl)phenyl phosphinate, anisoin, benzoin isobutyl ether, 4-benzoylbiphenyl, 2-benzyl-2-(dimethylamino)-4'-morpholinobutyrophenone, 4,4'-dimethylbenzil, diphenyl(2,4,6-trimethylbenzoyl)phosphine oxide /2-hydroxy-2-methylpropiophenone 50/50 blend, 4'-ethoxyacetophenone, 2,4,6-trimethylbenzoyldiphenylphosphine oxide, 3'-hydroxyacetophenone, 4'-hydroxyacetophenone, methybenzoylformate, 4'-phenoxyacetophenone, polymeric derivatives thereof and combinations thereof.

Preferred radical photoinitiators are acetophenones, α-hydroxy acetophenones, phosphine oxides and acylphosphine oxides, more preferably acetophenones and acylphosphine oxides.

In particular, the radical photoinitiator may be selected from an acetophenone such as SpeedCure^{®} BKL (2,2-dimethoxy-1,2-phenylacetophenone); an acylphosphine oxide such as SpeedCure^{®} XKM (ethyl phenyl(2,4,6-trimethylbenzoyl)phosphinate), SpeedCure^{®} BPO (phenyl bis(2,4,6-trimethylbenzoyl)-phosphine oxide), SpeedCure^{®} TPO (2,4,6-trimethylbenzoyldiphenylphosphine oxide) or SpeedCure^{®} TPO-L (ethyl (2,4,6-trimethylbenzoyl)phenyl phosphinate); and mixtures thereof.

The amount of radical photoinitiator may be varied as may be appropriate depending on the radical photoinitiator(s) selected, the amounts and types of polymerizable species cured with the process, the radiation source and the radiation conditions used, among other factors. Typically, however, the amount of radical photoinitiator may be from 0% to 10%, for example 0.05% to 10%, in particular 0.1% to 5%, more particularly 0.5 to 2%, by weight of radical photoinitiator based on the total weight of the one or more ethylenically unsaturated compounds. For example, the amount of radical photoinitiator may be from 0.01% to 5%, from 0.02% to 3%, from 0.05 to 2%, from 0.1 to 1.5% or from 0.2 to 1%, by weight based on the total weight of the one or more ethylenically unsaturated compounds. In another example, the amount of radical photoinitiator may be from 1% to 5%, from 1.5% to 5%, from 2 to 5%, from 2.5 to 5% or from 3 to 5%, by weight based on the total weight of the one or more ethylenically unsaturated compounds.

### Radical stabilizer

The process of the invention may further comprise mixing a radical stabilizer with said compound of formula (I) and said one or more ethylenically unsaturated compounds. The radical stabilizer is a free radical polymerization inhibitor. It is preferably selected from the group consisting of hindered phenolic or polyphenolic compounds; preferably from hydroquinone, hydroquinone monomethyl ether, mono-tertiary-butyl hydroquinone, 2,5-ditertiary-butyl-hydroquinone, p-methoxyphenol, hydroxyanisole, butylated hydroxyanisole, hydroxyanisole butyl ether, 2,6-di-tert-butyl-p-cresol, 2,2'-methylene-bis-(6-tert-butyl-4-methylphenol), p-tert-butyl catechol, 1,3,5-trimethyl-2,4,6-tris(3,5-di-tert-butyl-4-hydroxybenzyl) benzene, hydroxytoluene butyl ether, and mixtures thereof; more preferably from hydroquinone monomethyl ether, 2,2'-methylene-bis-(6-tert-butyl-4-methylphenol), and mixtures thereof.

The amount of radical stabilizer may be from 0.01 to 0.7 % by weight, preferably from 0.01 to 0.5% by weight, more preferably from 0.01 to 0.4 % by weight, based on total weight of the one or more ethylenically unsaturated compounds.

### Solvent

Advantageously, the process of the invention may be carried out in the absence of solvent, i.e., in the absence of non-reactive volatile substances.

However, in certain other embodiments of the invention, the process of the invention may further comprise mixing one or more solvents with said compound of formula (I) and said one or more ethylenically unsaturated compounds . The one or more organic solvents may be non-reactive organic solvents. In various embodiments, the solvent(s) may be relatively volatile, e.g., solvents having a boiling point at atmospheric pressure of not more than 150° C. In other embodiments, the solvent(s) may have a boiling point at atmospheric pressure of at least 40°C.

The process may be carried out in the presence of less than 10% or less than 5% or even 0% of non-reactive solvent, based on the total weight of the one or more ethylenically unsaturated compounds

Suitable solvents may include, for example, organic solvents such as: ketones; esters; carbonates; alcohols; aromatic solvents such as xylene, benzene, toluene, and ethylbenzene; alkanes; glycol ethers; ethers; amides; as well as combinations thereof.

### Other additives

The process of the invention may further comprise mixing one or more other additives with said compound of formula (I) and said one or more ethylenically unsaturated compounds. Such other additives include, but are not limited to, free radical chain transfer agents, antioxidants, ultraviolet absorbers, light blockers, photostabilizers, foam inhibitors, flow or leveling agents, fillers, colorants, fluorophores, pigments, dispersants (wetting agents), slip additives, plasticizers, thixotropic agents, matting agents, impact modifiers, adhesion promoters, thermoplastics such as acrylic resins that do not contain any free radical-polymerizable functional groups, waxes or other various additives, including any of the additives conventionally utilized in the coating, sealant, adhesive, molding, 3D printing or ink arts.

### Compound of formula (I') or (1a) to (1h)

The invention also relates to a compound of formula (I'): 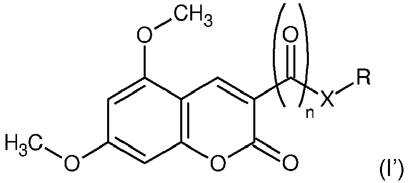 wherein
n is 1 or 2;
X represents S, Se or Te;
R is a (C₅-C₁₂)aryl optionally substituted by one or more groups chosen from -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{d} and Rₑ identical or different are independently chosen from H and linear or branched (C₁-C₂₀)alkyl, and R_{c} is chosen from linear or branched -(C₁-C₂₀)alkyl and (C₅-C₁₂)aryl optionally substituted by one or more of -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched-S(C₁-C₂₀)alkyl, -NR_{d}Rₑ, -NO₂ and -CN.

The preferred embodiments for n, X and R disclosed above for the compound of formula (I) equally apply to the compound of formula (I').

The invention also related to a compound of formula (1a) to (1h):

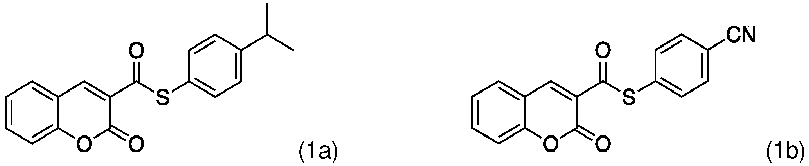

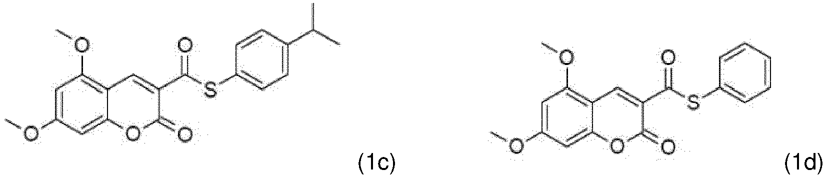

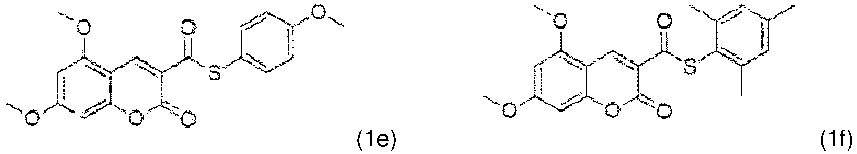

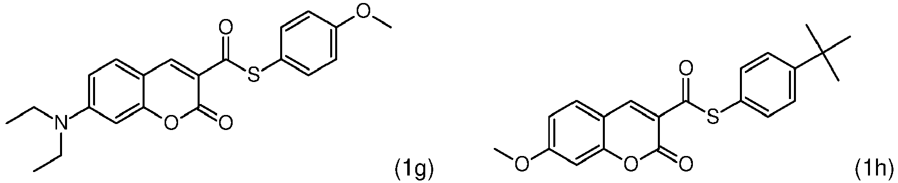

### Uses

The invention also relates to the use of a compound of formula (I') or a compound of formula (1a) to (1h) as a photoinitiator. In particular, said compounds may be used as a photoinitiator having photobleaching properties.

Unexpectedly, the compounds of formulae (I') and (1a) to (1h) do not suffer from oxygen inhibition.

The compounds of formulae (I') and (1a) to (1h) may be used as a photoinitiator that is activable by irradiation with a LED light source, preferably a LED light source having a maximum output wavelength in the range of 250 to 550 nm, in particular 250 to 460 nm, more particularly 340 to 430 nm.

The photoinitiators of the present invention may have potential applications in photocurable inks, coatings, adhesives, advanced materials, electronics and in additive manufacturing (3D printing).

The invention particularly relates the use of a compound of formula (I') or (1a) to (1h) as a photoinitiator for curing in air formulations comprising one or more ethylenically unsaturated compounds which may be polymerized by free radical, anionic and hybrid anionic/free radical polymerization. Said ethylenically unsaturated compounds may be as described above for the process.

The compounds of formulae (I') and (1a) to (1h) advantageously show high cure speeds, low yellowing and/or photobleaching characteristics and high thermal stability in formulations. The yellowing characteristic can be measured by the Colour index 'b' value on cured films.

### Process for the preparation of a compound of formula (I')

The invention also relates to a process for the preparation of a compound of formula (I') or a compound of formula (1a) to (1h) as defined above, the process comprising reacting a compound of formula (III) with a compound of formula (IV):

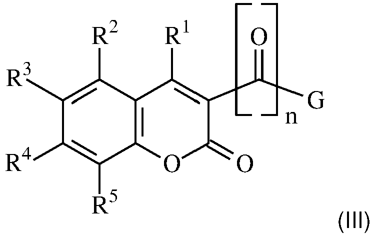

H―X―R (IV)

wherein
n, R¹, R², R³, R⁴,R⁵, R and X are as defined above;
G is OH, a halogen atom or -O-C(=O)-J;
J is alkyl or aryl, in particular tert-butyl.

Typically, the reaction of a compound of formula (III) where G is halogen with a compound of formula (IV) may be carried out in an organic solvent, preferably a non-polar aprotic solvent such as THF, in the presence of a base, preferably a weak organic base such as triethylamine.

The reaction may be conducted in a wide range of temperature, from -20°C to the reflux temperature of the reactional mixture.

According to an embodiment, the process of the invention may also comprise the step of preparing intermediates of formula (III).

According to an embodiment, the compound of formula (III) where G is a halogen atom may typically be prepared by halogenation of the corresponding compound of formula (III) where G is OH.

As an illustrative example, the compound (III) where G is CI may be obtained by reacting the compound (III) where G is OH with thionyl chloride.

According to another embodiment, the compound of formula (III) where n=2 may typically be prepared by reacting a compound of formula (III) where n is 1 and G is CH₃ with an oxidizing agent such as selenium oxide (SeO₂) in the presence of a base, such as pyridine.

Generally, starting products (III) as well as reagents (IV) are commercially available or can be synthesized by application or adaptation of known procedures. For example, reagent (IV) may be a polythiol.

The process can comprise a further step of purifying the compound of formula (I') obtained for example by column chromatography.

The invention also relates to a process for the preparation of a compound of formula (I') or a compound of formula (1a) to (1h) as defined above, the process comprising reacting a compound of formula (VI) with a compound of formula (IV):

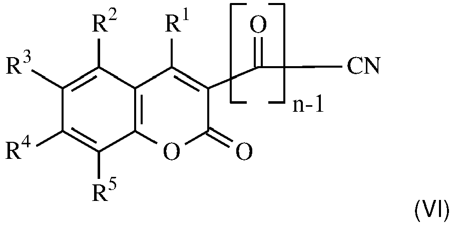

H―X―R (IV)

wherein n, R¹, R², R³, R⁴, R⁵, R and X are as defined above.

Typically, the reaction of a compound of formula (VI) with a compound of formula (IV) may be carried out in an organic solvent, preferably a non-polar organic solvent such as benzene, toluene or a dialkylether, in the presence of an acid, preferably a strong acid such as HCl.

The invention also relates to a process for the preparation of a compound of formula (I') or a compound of formula (1a) to (1h) as defined above, the process comprising reacting a compound of formula (VI) with hydrogen sulfide (H₂S) and a compound of formula (VII):

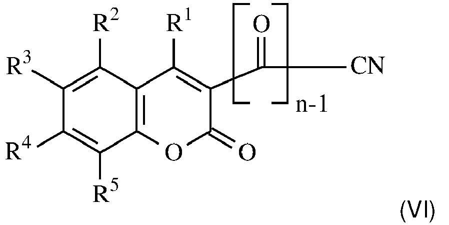

Hal―R (VII)

wherein
n, R¹, R², R³, R⁴, R⁵ and R are as defined above;
Hal is a halogen atom chosen from Cl, Br, I.

Typically, the reaction of a compound of formula (VI) with hydrogen sulfide and a compound of formula (VII) may be carried out in water or an organic solvent, preferably a polar organic solvent such as dimethylformamide or acetone, in the presence of a base, preferably a weak organic base such as pyridine.

The reaction may be conducted in a wide range of temperature, from -20°C to the reflux temperature of the reactional mixture, preferably at 50-80 °C.

The examples and figures hereafter illustrate the invention.

### Figures

Figure 1 shows the UV absorption of thioester coumarin based photoinitiators in dichloromethane at a concentration of 10⁻⁴ M in dichloromethane.
Figure 2 illustrates the photolysis of thioester coumarin based photoinitiators in dichloromethane at a concentration of 10⁻⁴ M at 385 nm LED exposure.
Figure 3 illustrates the conversion rates of photopolymerization using compared photoinitiators (coumarins with aliphatic or aromatic substituent, and the state-of-the-art Speedcure TPO-L) in TMPTA at 385 nm.
Figure 3a shows the reactivity of the photoinitiators under laminate.
Figure 3b shows the reactivity of the photoinitiators under air.
Figure 4 illustrates the photobleaching ability of compared photoinitiators (coumarins with aliphatic or aromatic substituent, and the state-of-the-art Speedcure TPO-L) in SR454 (ethoxylated TMPTA) at 385 nm.
Figure 4a shows the reactivity of the photoinitiators under laminate.
Figure 4b shows the reactivity of the photoinitiators under air.

### Examples

### Example 1: Preparation of compounds of formula (I)

### Example 1a: S-(4-isopropylphenyl) 2-oxo-2H-chromene-3-carbothioate

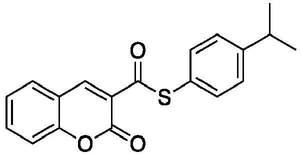

Coumarin-3-carboxylic acid (190 mg, 1 mmol, 1 eq.) was dissolved in thionyl chloride (1 mL) in a round bottom flask and stirred at 80°C for 4 hours under nitrogen. The mixture was evaporated to dryness to give the crude acyl chloride coumarin. This solid was dissolved in anhydrous THF (2 mL). Meanwhile, anhydrous THF (2 mL) was placed in a 3-necked flask, flushed well with nitrogen and cooled at 0°C. Triethylamine (0.42 mL, 3 mmol, 3 eq.), 4-isopropylbenzene thiol (0.31 mL, 2 mmol, 2 eq.) and finally the acid chloride were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-(4-isopropylphenyl) 2-oxo-2H-chromene-3-carbothioate as a light yellow solid (160 mg, 49%).

### Example 1b: S-(4-cyanophenyl) 2-oxo-2H-chromene-3-carbothioate

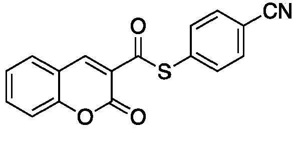

Coumarin-3-carboxylic acid (190 mg, 1 mmol, 1 eq.) was dissolved in thionyl chloride (1 mL) in a round bottom flask and stirred at 80°C for 4 hours under nitrogen. The mixture was evaporated to dryness to give the crude acyl chloride coumarin. This solid was dissolved in anhydrous THF (2 mL). Meanwhile, anhydrous THF (2 mL) was placed in a 3-necked flask, flushed well with nitrogen and cooled at 0°C. Triethylamine (0.42 mL, 3 mmol, 3 eq.), 4-thiobenzonitrile (270 mg, 2 mmol, 2 eq.) and finally the acid chloride were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-(4-cyanophenyl) 2-oxo-2H-chromene-3-carbothioate as a light yellow solid (219 mg, 71%).

### Example 1c: S-(4-iscpropylphenyl) 5,7-dimethoxy-2-oxo-2H-chromene-3-carbothioate

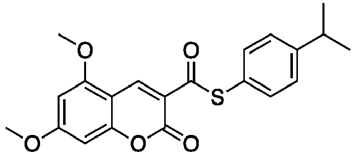

5,7-dimethoxy-2-oxo-2H-chromene-3-carboxylic acid (102 mg, 0.41 mmol, 1 eq.) was dissolved in thionyl chloride (0.41 mL) and DMF (2 drops) in a round bottom flask and stirred at 80°C for 2 hours under nitrogen. The mixture was evaporated to dryness to give the crude acyl chloride coumarin. This solid was dissolved in anhydrous THF (1 mL). Meanwhile, anhydrous THF (2 mL) was placed in a 3-necked flask, flushed well with nitrogen and cooled at 0°C. Triethylamine (0.17 mL, 1.23 mmol, 3 eq.), 4-isopropylbenzene thiol (0.13 mL, 0.82 mmol, 2 eq.) and the acid chloride were added and the mixture was gradually warmed to room temperature and stirred overnight. The reaction was then slowly quenched into ice/water, the solid obtained was filtered and washed with water. The solid was then charged in a flask and suspended in dichloromethane (50 mL) with stirring. The organic phase was extracted with water (50 mL) / 1M HCl (10 mL) (pH 3) then with a solution of NaHCOs (50 mL) and finally with water (50 mL). The product was dried with MgSO₄, filtered and the solvent was removed under reduced pressure to afford a solid. Diethyl ether (25 mL) was added to the solid and stirred for 3 hours to remove the residual thiol. The solid was filtered, washed with diethyl ether (5 mL) and dried to afford S-(4-isopropylphenyl) 5,7-dimethoxy-2-oxo-2H-chromene-3-carbothioate as a yellow solid (52 mg, 33%).

### Example 2: Properties of the exemplified compounds

### 2.1 UV absorption

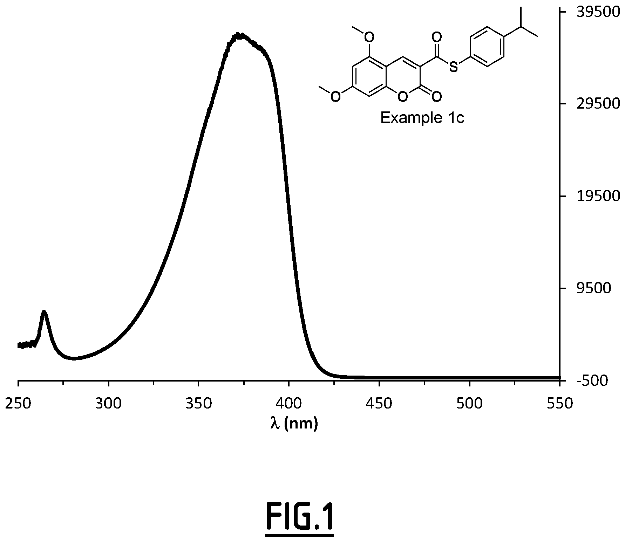

The UV absorption of thioester coumarin based photoinitiator of Example 1c in dichloromethane at a concentration of 10⁻⁴ M in dichloromethane is illustrated in Figure 1.

Figure 1 shows that the coumarin based photoinitiator with aromatic substituent has good absorbance up to 425 nm. This makes it suitable and reactive enough to be used in formulations cured under LED conditions.

### 2.2 Photolysis

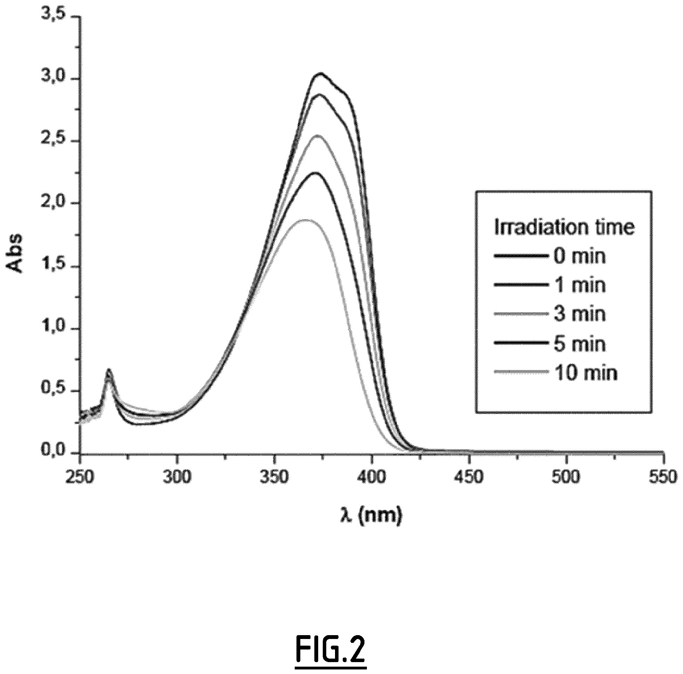

The results of the photolysis of the thioester coumarin based photoinitiator of Example 1c in dichloromethane at a concentration of 10⁻⁴ M at 385 nm LED exposure is illustrated in Figure 2 .

Figure 2 shows that the coumarin based photoinitiator with aromatic substituent is able to undergo substantial photocleavage after irradiation under LED wavelengths.

### Example 3: Curing performances of compounds of example 1

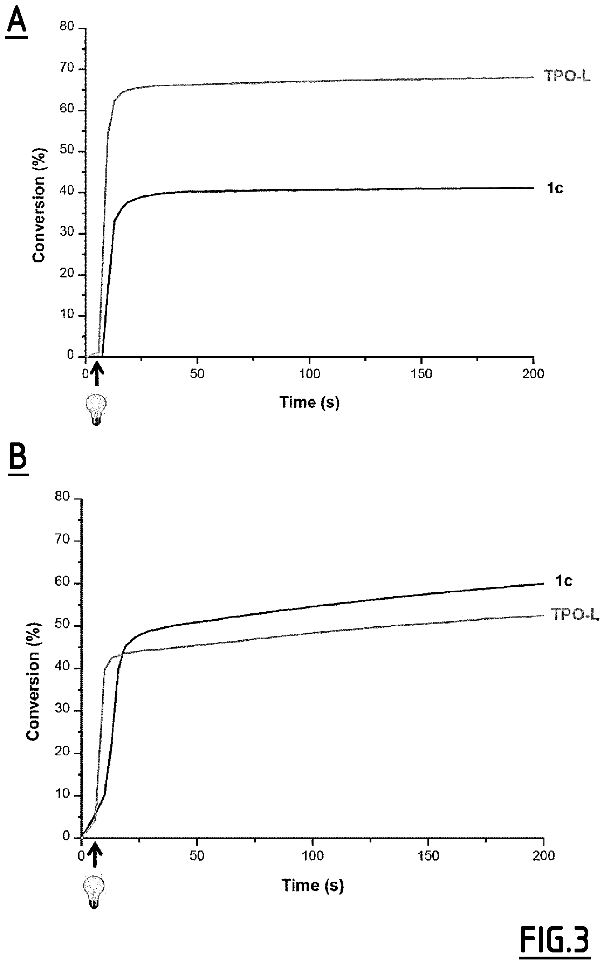

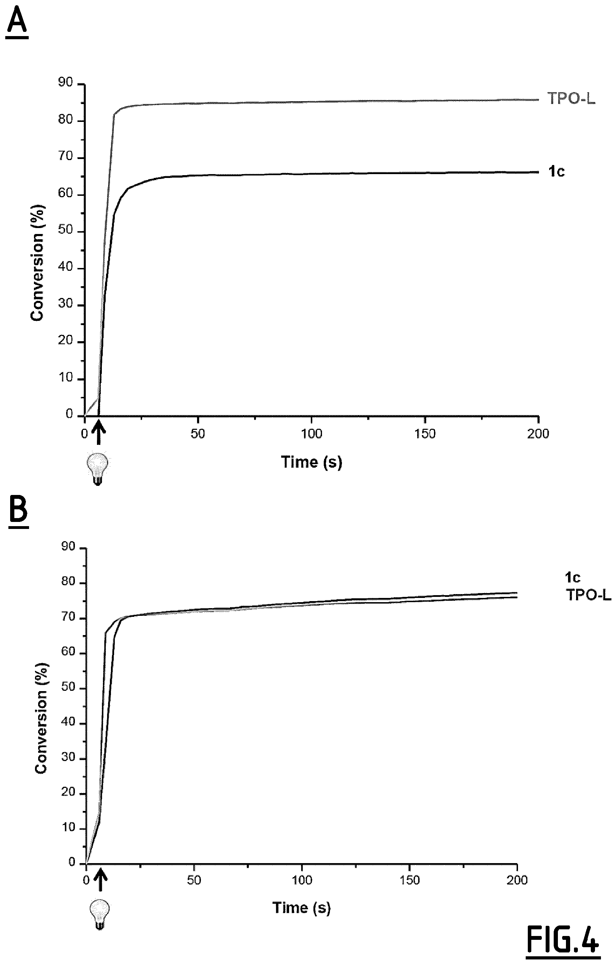

The curing performance of the thioester coumarin based photoinitiator of Example 1c and state-of-the-art Speedcure TPO-L using real time FTIR measurement is illustrated in Figures 3 and 4.

The photoinitiators were dissolved at 1% in trimethylolpropane triacrylate (TMPTA) or ethoxylated trimethylolpropane triacrylate (SR454) at room temperature and were stirred overnight.

The photosensitive formulations were deposited on a polypropylene film and the sample thickness was controlled using a calibrated bar (24 µm, 12 µm or 6 µm). The samples were polymerized under air or in laminate (between two polypropylene films) using a LED at 385 nm (I₀ = 160 mW.cm⁻²) exposure at room temperature.

For radical polymerization, the evolution or the double bond content of the acrylate function was continuously followed by real-time Fourier Transform Infrared (FTIR) spectroscopy (JASCO FTIR 6600) at about 1630 cm⁻¹ for thin samples.

The conversion rates were measured over time. Results are illustrated in Figures 3 and 4.

Figure 3a shows the reactivity of Example 1c compared with Speedcure TPO-L in TMPTA under laminate at 385 nm. Figure 3b shows the reactivity of Example 1c compared with Speedcure TPO-L in TMPTA under air at 385 nm.

In laminate, TPO-L is the best performer with the highest acrylate conversion, followed by the aromatic coumarin.

However, under air (Figure 3b), the coumarin of the invention (Example 1c) unexpectedly outperforms TPO-L.

TPO-L decreases acrylate conversion when moving from laminate to under air (i.e it is inhibited by oxygen) whereas the coumarin of the invention shows the opposite effect.

Figures 4a-4b show the same information as Figures 3a-3b using SR454 (ethoxylated TMPTA) instead of TMPTA as the ethylenically unsaturated compound.
The ethoxylation of the TMPTA does reveal some differences. At all wavelengths in laminate TPO-L is the best performer with the highest acrylate conversion, followed by the aromatic coumarin of Example 1c. Again under air, TPO-L acrylate conversion is decreased, whereas the aromatic coumarin of Example 1c has the highest acrylate conversion after 200 s of irradiation.

The L*, a*, b* values of the photosensitive formulations were determined before and after photopolymerization using a spectrophotometer from Thorlabs in transmission experiments. The photobleaching ability of each of the coumarins vs TPO-L at 385 nm was also studied. The b* values are detailed in Table 1 below.

| | | **Laminate** | | **Air** | |
|---|---|---|---|---|---|
| | | **Before polymerization** | **After polymerization** | **Before polymerization** | **After polymerization** |
| **TMPTA** | **Example 1c** | 7.21 | 5.71 | 10.02 | 7.58 |
| | **TPO-L** | 5.34 | 5.55 | 5.53 | 5.43 |
| **SR454** | **Example 1c** | 11.24 | 5.77 | 8.46 | 5.60 |
| | **TPO-L** | 5.29 | 5.15 | 5.48 | 5.13 |

As can be seen from these results, the photoinitiators according to the invention are effective photoinitiators under LED lamp conditions which are not inhibited by oxygen, contrary to the state-of-the-art Speedcure TPO-L. Further, they exhibit photobleaching properties.

## Claims

1. A process for curing one or more ethylenically unsaturated compounds, said process comprising:
- mixing one or more ethylenically unsaturated compounds with a compound of formula (I): 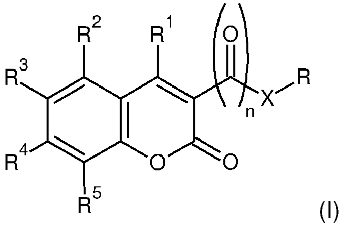 and
- irradiating said mixture with at least one light source under air;
wherein in Formula (I):
n is 1 or 2;
X represents S, Se or Te;
each R¹, R², R³, R⁴ and R⁵ is independently chosen from the group consisting of H, -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ; or two adjacent R², R³, R⁴ and R⁵ groups may form, with the carbon atoms to which they are attached, a 3 to 12 membered aliphatic ring or a 5 to 12 membered aromatic ring; wherein Rₐ, R_{b}, R_{d} and Rₑ identical or different are independently chosen from H and linear or branched (C₁-C₂₀)alkyl; and R_{c} is chosen from linear or branched -(C₁-C₂₀)alkyl and (C₅-C₁₂)aryl optionally substituted by one or more of -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -NR_{d}Rₑ, -NO₂ and -CN;
R is a (C₅-C₁₂)aryl optionally substituted by one or more groups chosen from -OH, -SH, halogen, linear or branched -(C₁-C₂₀)alkyl, linear or branched -(C₁-C₂₀)haloalkyl, -(C₁-C₂₀)perfluoroalkyl, linear or branched -(C₂-C₂₀)alkenyl, linear or branched -O(C₁-C₂₀)alkyl, linear or branched -S(C₁-C₂₀)alkyl, -(C₃-C₁₂)cycloalkyl, -(C₅-C₁₂)aryl, -O(C₅-C₁₂)aryl, -S(C₅-C₁₂)aryl, -(C₁-C₂₀)-alkylene-(C₅-C₁₂)aryl, -(C₅-C₁₂)-arylene-(C₁-C₂₀)alkyl, -NO₂, -CN, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined above.

2. The process according to claim 1, wherein R¹ is H and R², R³, R⁴ and R⁵ are independently chosen from the group consisting of H, linear or branched -O(C₁-C₂₀)alkyl and -NR_{d}Rₑ wherein R_{d} and Rₑ are independently a linear or branched -(C₁-C₂₀)alkyl.

3. The process according to claim 1 or 2, wherein R², R³ and R⁵ are H and each of R¹ and R⁴ is independently a linear or branched -O(C₁-C₆)alkyl, preferably methoxy.

4. The process according to any one of claims 1 to 3, wherein R is a phenyl or a naphthyl, said phenyl or naphthyl being optionally substituted by one or more groups selected from halogen, -CF₃, -NO₂, -CN, (C₅-C₁₂)aryl, linear or branched -(C₁-C₂₀)alkyl group, linear or branched -O(C₁-C₂₀)alkyl group, linear or branched -S(C₁-C₂₀)alkyl group, -C(=O)ORₐ, -OC(=O)R_{b}, -C(=O)R_{c}, -NR_{d}Rₑ and -C(=O)NR_{d}Rₑ, wherein Rₐ, R_{b}, R_{c}, R_{d} and Rₑ are as defined claim 1, preferably Rₐ, R_{b}, R_{c}, R_{d} and Rₑ are independently a linear or branched (C₁-C₂₀)alkyl.

5. The process according to any one of claims 1 to 4, wherein X is S.

6. The process according to any one of claims 1 to 5, wherein n is 1.

7. The process according to any one of claims 1 to 6, wherein said irradiating step is conducted with at least one LED light source, preferably a LED light source having a maximum output wavelength in the range of 250 to 550 nm, in particular 250 to 460 nm, more particularly 340 to 430 nm.

8. The process according to any one of claims 1 to 7, wherein the one or more ethylenically unsaturated compounds comprise at least one of a cyanoacrylate-functionalized compound, a (meth)acrylate-functionalized compound and mixtures thereof.

9. The process according to any one of claims 1 to 8, which further comprises mixing an anionic synergist, in particular a metallocene, more particularly a ferrocene with said compound of formula (I) and said one or more ethylenically unsaturated compounds.

10. A compound of formula (I'): 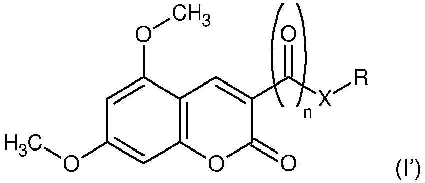 wherein n, X and R are as defined in any one of claims 1 and 4 to 6.

11. A compound of formulae (1a) to (1h):

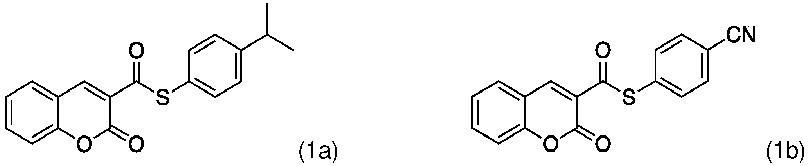

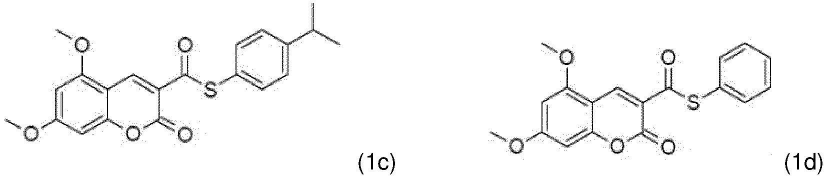

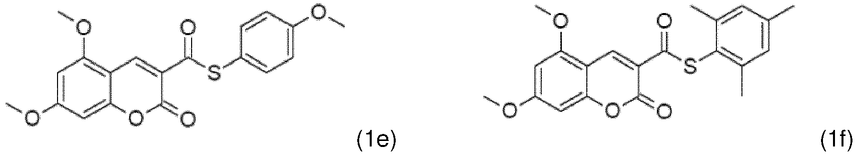

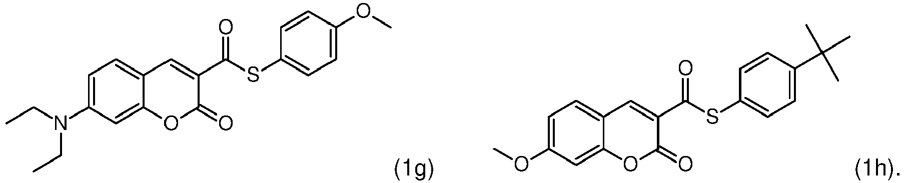

12. A process for the preparation of a compound of formula (I') according to claim 10 or a compound of formula (1a) to (1h) according to claim 11, said process comprising reacting a compound of formula (III) with a compound of formula (IV): 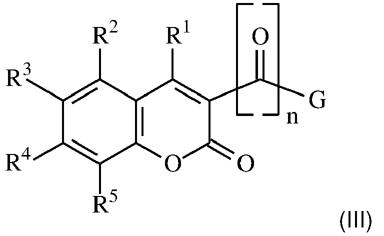
H―X―R (IV)
wherein
n, R¹, R², R³, R⁴, R⁵, R and X are as defined in claim 10 or 11;
G is OH, a halogen atom or -O-C(=O)-J;
J is alkyl or aryl, in particular tert-butyl.

13. A process for the preparation of a compound of formula (I') according to claim 10 or a compound of formula (1a) to (1h) according to claim 11, said process comprising reacting a compound of formula (VI) with a compound of formula (IV): 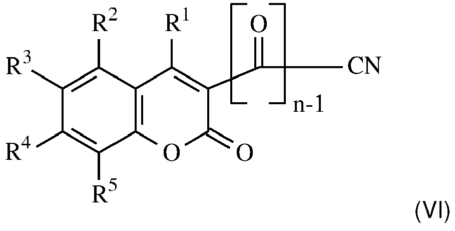
H―X―R (IV)
wherein n, R¹, R², R³, R⁴, R⁵, R and X are as defined in claim 10 or 11.

14. A process for the preparation of a compound of formula (I') according to claim 10 or a compound of formula (1a) to (1h) according to claim 11, said process comprising reacting a compound of formula (VI) with hydrogen sulfide (H₂S) and a compound of formula (VII): 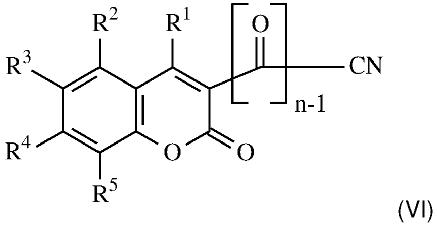
Hal―R (VII)
wherein n, R¹, R², R³, R⁴, R⁵ and R are as defined in claim 10 or 11;
Hal is a halogen atom chosen from C!, Br, I.

15. Use of a compound of formula (I') according to claim 10 or a compound of formula (1a) to (1h) according to claim 11, as a photoinitiator, in particular as a photoinitiator having photobleaching properties.
